Europäisches Patentamt

European Patent Office

Office européen des brevets

(11) Publication number: **0 255 233**
A2

(12) ## EUROPEAN PATENT APPLICATION

(21) Application number: 87305709.5

(22) Date of filing: 26.06.87

(51) Int. Cl.4: **C12N 15/00** , C07H 21/04 , C12N 1/18 , C12P 21/02

The applicant has filed a statement in accordance with Rule 28 (4) EPC (issue of a sample only to an expert). Accession number(s) of the deposit(s): FERM BP-1345.

(30) Priority: 30.06.86 JP 151809/86
20.08.86 JP 192639/86
23.02.87 JP 37869/87

(43) Date of publication of application:
03.02.88 Bulletin 88/05

(84) Designated Contracting States:
AT BE CH DE ES FR GB GR IT LI LU NL SE

(71) Applicant: Takeda Chemical Industries, Ltd.
27, Doshomachi 2-chome Higashi-ku
Osaka-shi Osaka, 541(JP)

(72) Inventor: Kikuchi, Masakazu
4-16, Higashitokiwadai 7-chome Toyono-cho
Toyono-gun Osaka 563-01(JP)
Inventor: Yoshimura, Koji
6-31, Zuiko 1-chome
Higashiyodogawa-ku Osaka 533(JP)
Inventor: Nakahama, Kazuo
15-59, Nishinokyo Nagaokakyo
Kyoto 617(JP)

(74) Representative: Lewin, John Harvey et al
Elkington and Fife High Holborn House 52/54
High Holborn
London WC1V 6SH(GB)

(54) **Production of human lysozyme.**

(57) A DNA sequence wherein a DNA segment which codes for hen egg white lysozyme signal peptide and which is synthesized based upon codon usage for yeast is bound to the 5' end of a DNA segment coding for human lysozyme, a cell transformed with the above DNA sequence, and a process for producing human lysozyme by cultivating the above cell are disclosed.

The DNA sequence makes it possible to provide a large amount of human lysozyme, which is useful as pharmaceuticals without side-effects.

EP 0 255 233 A2

## PRODUCTION OF HUMAN LYSOZYME

Background of the Invention

(a)Field of the Invention

This invention relates to recombinant DNA techniques for producing human lysozyme. More particularly, it relates to the expression. of the human lysozyme gene, recombinant plasmids, transformed cells, and their products.

(b)Decription of the Prior Art

Lysozyme is a comparatively small enzyme protein with a molecular weight of approx. l4,000. Lysozyme is distributed in living tissue and is considered to play a role as a defensive substance against bacterial infections by dissolving various bacteria. The lysozyme function is thought to involve hydrolysis of polysaccharide of the bacterial cell wall by $\beta$-glucosidase activity. A good deal of lysozyme is contained in hen egg white and lysozyme with high purity can be isolated therefrom comparatively easily. Lysozyme is thus added to cheeze, sausage and marine products for their preservation or used for the purpose of converting bovine milk to human maternal milk [Katsuya Hayashi and Taiji Imoto, "Lysozyme", Nankodo, Japan (l974)]. Further, as a medicinal agent, lysozyme is accepted as a hemostatic, anti-inflammation, tissue regeneration, anti-tumor etc. (see for instance "Saikin-no-Shinyaku" (New-Drugs in Japan) vol. 34, l07, Yakuji Nippo, Tokyo, Japan, l983) and it is also on sale as anti-inflammatory enzyme agent.

When the lysozyme derived from egg white is used for medical purposes, sensitive symptoms such as rash, redness often result. These are generally regarded as a side effect of the immune response to the presence of a foreign protein. To overcome the above-mentioned disadvantges, the present inventors began studying to establish a means for the mass production of human lysozyme.

Amino acid sequence of human lysozyme is known, and which consist of l30 amino acids ["Biochemical Data Book" l, l89 (l979), editted by Japan Biochemical Associa-tion]. On the basis of the amino acid sequence, a DNA fragment coding for the human lysozyme is chemically synthesized [Ikehara, M. et al, Chem. Pharm. Bull. 34, 2202 (l986)].

For the production of human lysozyme using a recombinant DNA technique, Muraki et al tried to express it in E. coli but failed to obtain active human lysozyme with such an expression system [Muraki, M. et al, Agric. Biol. Chem. 50, 7l3 (l986)].

An active human lysozyme has been found to be obtained by secretion using a yeast [Jigami et al, Summary of Japanese Agricultural Chemistry Association l986, p.343 (l986)]. But the yield is very low, i.e. only 600 µg/litre. According to the report by Jigami et al, a cDNA fragment coding for a signal peptide having information for extracellular secretion of a desired protein is used in combination with a DNA fragment coding for a human lysozyme for the expression of the human lysozyme. As the cDNA fragment coding for the signal peptide, a cDNA fragment (natural type) coding for a signal peptide of egg white lysozyme is used [The signal peptide has quite the same sequence as that disclosed in Pro. Natl. Acad. Sci. USA 77, 5759-5763 (l980)]. With such a combination, however, the lysozyme is secreted extracellularly from yeast cells in a very small amount as described above.

Summary of the Invention

The present inventors have made an intensive study on the production of active human lysozyme on a large scale utilizing a recombinant DNA technique and, as a result, have completed the present invention.

Although the human lysozyme gene has not yet been cloned at present, the amino acid sequence of the human lysozyme has been determined. Thus, Ikehara et al have chemically synthesized a DNA coding for the human lysozyme on the basis of the amino acid sequence [Ikehara, M. et al (supra)].

It is therefore possible to produce the human lysozyme in a large amount by recombinant DNA technique with the use of the chemically synthesized DNA.

As a DNA fragment coding for a signal peptide to be used with the DNA fragment coding for the human lysozyme, there may be mentioned a cDNA fragment (natural type) coding for the above-described hen egg white lysozyme signal peptide. In consideration of expression in a yeast, a DNA fragment coding for hen egg white lysozyme signal peptide has been prepared by using preferentially codons which are used highly frequently in yeast. With such a DNA fragment, the desired object, namely obtaining human lysozyme in a large amount, has been found to be increasingly accomplished. Codon usage is reported in The Journal of Biological Chemistry 257 , 3026-303l (l982). Examples of preferable codons for yeast are given below in terms of codons for RNA.

| Amino acid | Codon | | Amino acid | Codon | Amino acid | Codon |
|---|---|---|---|---|---|---|
| Ala | GCU, | GCC | His | CAC | Tyr | UAC |
| Ser | UCU, | UCC | Glu | GAA | Cys | UGU |
| Thr | ACU, | ACC | Gly | GGU | Asn | AAC |
| Val | GUU, | GUC | Gln | CAA | Pro | CCA |
| Ile | AUU, | AUC | Lys | AAG | Met | AUG |
| Asp | GAC | | Leu | UUG | Trp | UGG |
| Phe | UUC | | Arg | AGA | | |

Brief Description of the Drawings:

Fig. l is a diagram showing a method for the synthesis of the human lysozyme gene by collecting and ligating oligonucleotides;

Fig. 2 shows a DNA sequence of the Taql-Xhol fragment of the human lysozyme gene;

Fig. 3 shows a DNA sequence prepared according to the present invention on the basis of egg white lysozyme signal peptide;

Fig. 4 shows a scheme for the construction of a human lysozyme secretion plasmid;

Fig. 5 shows the results of immuno blott assay to confirm the production of human lysozyme by the present invention;

Fig. 6 is a graph showing the relationship between absorbance and bacteriolytic activity of the expressed human lysozyme according the present invention which was purified by and· eluted from CM-Toyopearl 650C.

Fig. 7 shows the results of SDS-PAGE and staining of the peak fraction of Fig. 6 and the comparative materials such as human lysozyme standard sample; and

Fig. 8 shows the absorbance at a wavelength of 280 nm of the eluted human lysozyme from reversed phase HPLC after purification by CM-Toyopearl 650C.

Description of the Preferred Embodiment

In preparing the synthetic gene according to Ikehara et al., DNA sequence is selected with the consideration of the following points:

i) The most acceptable codons in yeast which is considered suitable for expression of synthetic gene are used.

ii) A specific recognition site of restriction enzyme (Xba I in this case) is made to serve as a marker for cloning or to fascilitate reconstruction of the gene after constructing an expression vector,

iii) Sequences which are self-complementary, or complementary with other sequence (except proper combination) in either strand or both strands are avoided.

The human lysozyme gene can be chemically synthesized as described above.

It is necessary to insert the synthetic gene in a proper vector for sub-cloning to enrich it. Though any kind of vector may be used so far as this gene can be inserted, Escherichia coli vector pACYCI77, E-scherichia coli-yeast shuttle vector pPHOI7, pGLD906 and pGLD906-I (Japanese Patent Unexamined Publication No.6I-4399I) and Bacillus subtilis vector pTEX20I [Yoshimura, K. et al, Appl. Microbiol. Biotechnol. 23, 250 (I986)] are cited as specific examples.

Various host organisms are transformed by using vectors containing synthetic genes thus obtained. The method of transformation itself is well known, but when Escherichia coli is used as a host organism, transformation is performed by the method of Cohen et al., [Cohen, S. N. et al., Proc. Natl. Acad. Sci. USA, 69, 2II0 (I972)], when yeast is used as a host organism, by the method of Hinnen et al., [Hinnen A et al., Proc. Natl. Acad. Sci. USA, 75, I927 (I978)] and when Bacillus subtilis is used as a host organism, by protoplast method [Chang, S. & Cohen S. N. Gen. Genet., I68, III (I979)] or competent method [Dubnau, D. & Abelson, R. D., J. Mol. Biol. 56, 209 (I97I)], respectively. As host organisms, for instance, E. coli 294, E. coli W3II0, E. coli C600, Saccharomyces cerevisiae AH22R⁻, B. subtilis IAI, B. subtilis IA339, B. subtilis IA340 may be used.

Then, in order to express the gene, the plasmid DNA in which the gene is inserted is first isolated from the transformants by for example, an alkaline extraction method [Birnboim, H. C. & Doly, J.:Nucleic Acids Res., 7, I5I3 (I979)]. By treating plasmid DNA thus obtained with proper restriction enzyme the inserted genes can be cut out and can be isolated by agarose gel electrophoresis or poly-acrylamide gel electrophoresis. All of these processes are well known and they are mentioned in literature in detail [see for example Molecular Cloning (I982), Cold Spring Harbor Laboratory]. The isolated gene is linked downstream from a promoter and signal peptide-coding region in a proper expression vector in the correct direction to construct an expression plasmid.

As the signal peptide, there may be mentioned a natural type hen egg white signal peptide. As a preferred DNA coding for the signal peptide, there may be mentioned a DNA chemically synthesized on the basis of codon usage for yeast. Although suitable codons of the chemically synthesized DNA are those which are most frequently used in yeast, codons in lower frequency may be used for the praparation of restriction enzyme recognition site(s). The DNA may be chemically synthesized, for example, by the method of Crea et al [Crea, R. et al, Proc. Natl. Acad. Sci. USA 75, 5765 (I978).

A DNA fragment wherein a DNA segment which codes for signal peptide is bound to the 5′-end of a DNA segment coding for human lysozyme may be prepared by linking a DNA fregment coding for the signal peptide to 5′-end of the DNA segment coding for human lysozyme, as mentioned previously.

The DNA fragment can also be prepared (a) by linking a DNA segment coding for signal peptide and a portion of N-terminus of human lysozyme with a DNA segment coding for human lysozyme lacking the portion of N-terminus thereof, (b) by linking a DNA segment coding for signal peptide lacking a portion of C-terminus thereof with a DNA segment coding for the portion of C-terminus of signal peptide and for human lysozyme, or (c) by linking DNA segment coding for signal peptide lacking a portion of C-terminus thereof, DNA segment coding for the portion of C-terminus of signal peptide and for a portion of N-terminus of human lysozyme and DNA segment coding for human lysozyme lacking the portion of N-terminus thereof. A number of expression vectors are known in the art. Any of them can be used as long as they are usable for the expression of the gene. Illustrative of suitable expression vectors are pPHOI7, pGLD906, pGLD906-I (supra), pcDX [Okayama, H. & Berg, P., Mol. Cell. Biol. 3, 280 (I983) and pKSV-I0 (manufactured by Pharmacia Inc.).

As the promoter, PHO5 promoter, GLD promoter, PGK promoter, ADH promoter, PHO8I promoter, for example, may be used when a yeast is used as host. When an animal cell is used as host, SV40 early gene promoter, metallothionein promoter and heat shock promoter, for example, may be used as the promoter.

The utilization of an enhancer is also effective for the expression.

As the host to be used for the expression, there may be mentioned yeasts such as Saccharomyces cerevisiae, mouse L cells and Chinese hamster oocyte cells. Other eukaryotic cells may be used, however.

A method of transforming a yeast has been previously described. A method of transforming eukaryotic cells such as animal cells is described in, for example, "Proteins, Nucleic Acids and Enzymes "28(I983) "Introduction of Recombinant DNA Into Cells and Expression Thereof" (Kyoritsu Shuppan).

Using the thus obtained secretion plasmid, host cells are transformed, thereby to obtain a desired transformant. The transformant is then cultivated in any known manner.

A culture medium to be used for yeasts may be, for example, Burkholder minimum medium [Bostian, K.L. et al, Proc. Natl. Acad. Sci. USA 77, 4505 (I980), Amer, J. Bot. 30, 206 (I943)] or its modified media. The cultivation may be generally conducted at a temperature of I5-40°C, preferably 24-37°C for a period of I0-96 hours, desirably 24-72 hours, with aeration and/or agitation if necessary.

When a transformant for which a eukaryotic cell such as animal cell is used as host is cultured, Eagle's MEM [H. Eagle, Science 130, 432 (1959)], modified Eagle's medium of Dulbecoo [Orgad Larb & William J. Ritter, J. Biol. Chem. 258, 6043 (1983)], or the like may be used as a culture medium. The cultivation may be generally conducted at a temperature of 30-42°C, preferably 35-37°C for about 1-10 days.

After the completion of the cultivation, cells and the supernatant are separated by well known methods. To collect human lysozyme remaining within the cells, the cells are disrupted by any conventional method, such as disruption with ultrasonication or a French press, a mechanical disruption such as crushing and a disruption with lytic enzyme. Further if necessary, human lysozyme thus produced may be extracted by surface active agents such as Triton-X100 and deoxycholate. The human lysozyme in supernatant or extract thus prepared is purified by convenional protein purification methods such as salting out, isoelectric point precipitation, gel filtration, ion-exchange chromatography and high performance liquid chromatography (HPLC, FPLC, etc.) to obtain the desired human lysozyme.

A human lysozyme has medicinal activities such as anti-inflammation, hemostasis, tissue regeneration and anti-tumor and is used as an antiphlogistic enzyme agent for eye lotion or as an anticeptic agent for foods. When used for pharmaceutical purposes, the human lysozyme does not exhibit such side effects that are caused in the case of the hen egg white lysozyme due to immune response. Hitherto, however, the human lysozyme has been only obtained in a small amount.

The present invention makes it possible to provide a large amount of human lysozyme useful as pharmaceuticals as described above.

## Example

The present invention will be illustrated in more detail hereinbelow by way of Reference Examples and Examples. These examples are, however, not to be considered as limiting the scope of the invention.

Saccharomyces cerevisiae AH22R⁻/pGELI25 disclosed in Example 3 has been deposited with the Institute for Fermentation, Osaka, Japan (IFO) under the designation of IFO-10211 and also with Fermentation Redearch Institute, Agency of Industrial Science and Technology, Ministry of International Trade and Industry, Japan (FRI) under the designation of FERM P-8806 since June 13, 1986, and the deposit has been changed to the deposit according to Budapest Treaty and stored at FRI under the accession number of FERM BP-1345.

## Reference Example 1

### Construction of Cloning Vector pBR322X:

After 5µg of Escherichia coli vector pBR322 was reacted with 1.5 units of restriction enzyme Bal I in 40 µl of reaction buffer [10 mM Tris-HCl (pH7.5), 10 mM MgCl₂, 1 mM dithiothreitol] at 37°C for 5 hours, the reaction mixture was extracted with phenol and DNA was precipitated with ethanol in accordance with a conventional method. 50 ng of phosphorylated Xho I linker d[pCCTCGAGG] (New England Biolabs) was added to this DNA and they were ligated with each other by T4 DNA ligase according to a conventional method. With this reaction solution Escherichia coli DH1 was transformed and plasmids were extracted from thus obtained ampicillin-resistant and tetracycline-resistant colonies according to the alkaline extraction method (previously mentioned) to obtain plasmid pBR322X with XhoI site in place of Ball site.

## Reference Example 2

### Preparation of Human Lysozyme Gene Fragment Bearing TaqI site around N-terminus:

In the report of Ikehara et al (supra) the human lysozyme gene is prepared from the 52 oligonucleotide blocks shown in Table I.

## Table 1

| Upper Strand No. | | Lower Strand No. | |
|---|---|---|---|
| U1 | TCGAGATGAAGGTTT | L26 | TCGAGCTATTAAAC |
| U2 | TTGAGAGATGCGAAT | L25 | ACCACAACCTTGAAC |
| U3 | TAGCCAGAACTTTGAAG | L24 | GTATTGTCTGACATC |
| U4 | AGATTGGGTATGGAC | L23 | TCTATTTTGGCATCT |
| U5 | GGCTACCGTGGTATT | L22 | GTTTCTCCAAGCGAC |
| U6 | TCTTTAGCCAACTGG | L21 | CCAGGCTCTAATACCCTG |
| U7 | ATGTGTCTTGCTAAG | L20 | TGGGTCACGGACAAC |
| U8 | TGGGAATCCGGCTATAAC | L19 | TCTCTTAGCGCAGGC |
| U9 | ACTAGAGCTACCAAT | L18 | AACAGCATCAGCAAT |
| U10 | TACAACGCTGGCGAC | L17 | GTTGTCCTGAAGC |
| U11 | CGTTCTACAGACTATGG | L16 | AAAGCTGAGCAAGAT |
| U12 | TATTTTCCAAATTAACT | L15 | AAGTGACAGGCGTTGAC |
| U13 | CTAGATATTGGTG | L14 | GGCACCTGGAGTCTTGC |
| U14 | TAACGATGGCAAGACTC | L13 | CATCGTTACACCAATAT |
| U15 | GAGGTGCCGTCAACGCC | L12 | CTAGAGTTAATTTGG |
| U16 | TGTCACTTATCTTGC | L11 | AAAATACCATAGTCTGT |
| U17 | TCAGCTTTGCTTCAG | L10 | AGAACGGTCGCCAGC |
| U18 | GACAACATTGCTGAT | L9 | GTTGTAATTGGTAGC |
| U19 | GCTGTTGCCTGCGCT | L8 | TCTAGTGTTATAGCCG |
| U20 | AAGAGAGTTGTCCGT | L7 | GATTCCCACTTAGCAAG |
| U21 | GACCCACAGGGTATT | L6 | ACACATCCAGTTGGC |
| U22 | AGAGCCTGGGTCGCT | L5 | TAAAGAAATACCACG |
| U23 | TGGAGAAACAGATGC | L4 | GTAGCCGTCCATACC |
| U24 | CAAAATAGAGATGTC | L3 | CAATCTCTTCAAAGT |
| U25 | AGACAATACGTTCAAGG | L2 | TCTGGCTAATTCGCATC |
| U26 | TTGTGGTGTTTAATAGC | L1 | TCTCAAAAACCTTCATC |

6

In Table I, CGAGAGATGCGAAT in place of U2 and TCTGGCTAATTCGCATCTCT in place of L2 were synthesized as U2-taq and L2-taq respectively according to the report of Ikehara et al. Then using each fragment U2-taq, U3 to U26, L2-taq, L3 to L26, hybrid of oligonucleotide blocks was formed according to the report of Ikehara et al (Fig I). After each of these groups were linked according to the method stated in Example 2, both 5'-ends were enzymatically phosphorylated.

Reference Example 3

Sub-cloning of Fragment of Human Lysozyme Gene containing Taq I Site

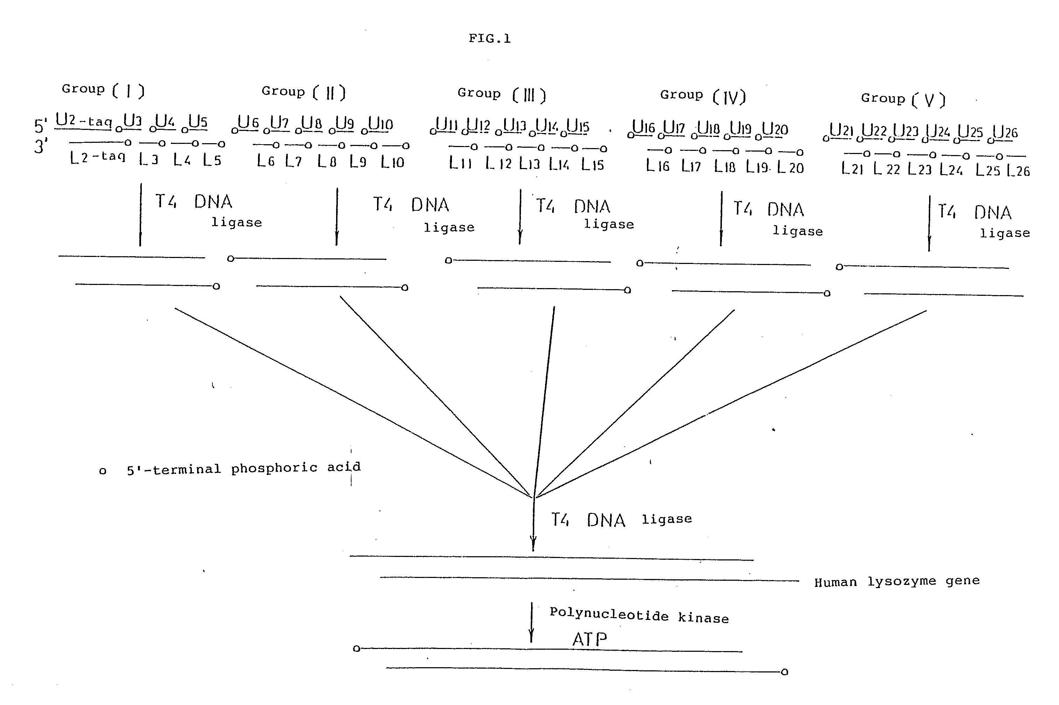

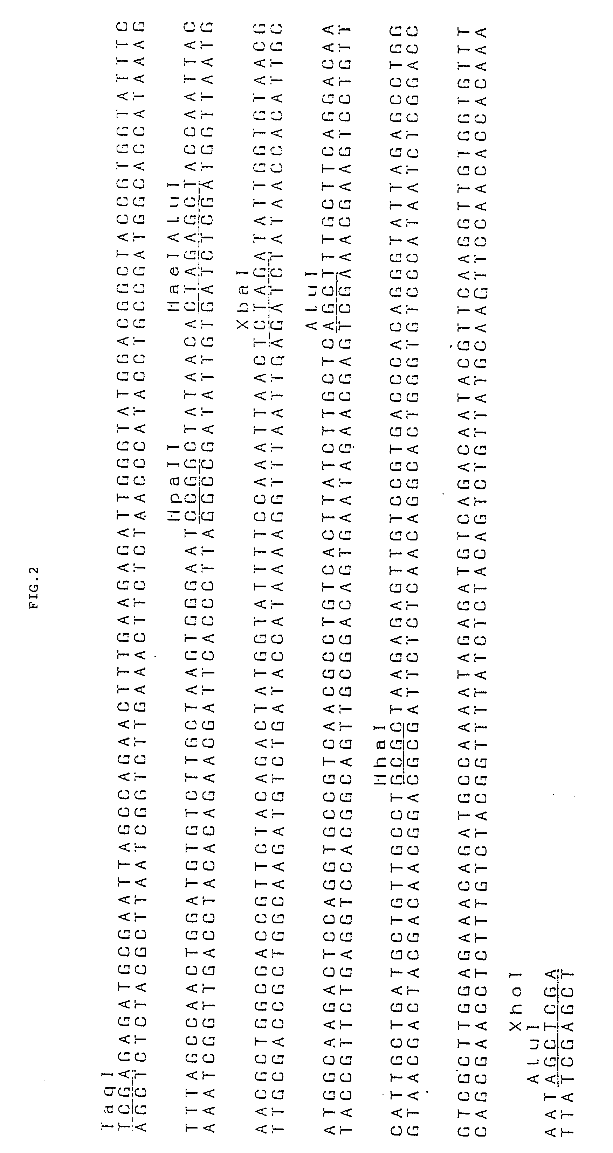

2.6 μg of plasmid pBR322X constructed at Reference Example I was reacted with 6 units of restriction enzyme XhoI and 6 units of restriction enzyme ClaI in 35 μl of a reaction solution [33 mM acetate buffer, pH 7.9, 66 mM potassium acetate, 10 mM magnesium acetate, 0.5 mM dithiothreitol, 0.01% BSA (bovine serum albumin)] at 37°C for I hour and the solution was deproteinized with phenol and precipitated with cold ethanol. This DNA (200 ng) was mixed with 100 ng of human lysozyme gene fragment prepared·in Reference Example 2 and allowed to react in 10 μl of a reaction solution [66 mM Tris-HCl (pH 7.6), 10 mM ATP, 10 mM spermidine, 100 mM MgCl₂, 150 mM DTT,·2 mg/ml BSA, 5 units of T4 DNA ligase] at 14°C overnight to be ligated with each other. Using this reaction solution Escherichia coli DHI was transformed according to the method by Cohen et al. Plasmids were isolated from transformants thus obtained according to the alkaline extraction method (previously mentioned). Their molecular weight and cleavage pattern with restriction enzymes were examined and pLYS221 in which the human lysozyme gene fragment was inserted was obtained. As the result of isolating EcoRI-XhoI fragment of pLYS221 and determining its base sequence in accordance with dideoxynucleotide chain termination method, TaqI - XhoI fragment of human lysozyme gene was obtained as shown in Figure 2 exactly assumed.

This sequence codes for the fourth Glu to the 130th Val of amino acid sequence of human lysozyme.

Reference Example 4

Preparation of Antibody Against Human Lysozyme:

Human lysozyme solution [500 μg of human lysozyme (Sigma), 500 μl of 0.85% NaCl, 500 μl of Freund's complete adjuvant] was injected subcutaneously at five places of each of three New Zealand white rabbits weighing 2.5 kg (the first immunization). Two weeks after the first immunization human lysozyme solution [500μg of human lysozyme (Sigma), 500 μl of 0.85% NaCl, 500 μl of Freund's incomplete adjuvant] was injected subcutaneously, in the same manner as the first immunization (the second immunization). Another two weeks later the third immunization was conducted in the same manner as the second immunization. One week after the third immunization, about 50 ml of blood per rabbit was collected from the ear of each rabbit. The blood was allowed to stand and the obtained supernatant was centrifuged at 3000 rpm for 10 minutes. The supernatant was used as antiserum.

Reference Example 5

Purification of Anti-Human Lysozyme Antibody

(NH₄)₂SO₄ was added at 5°C to the above-mentioned antiserum up to 40% saturation and the precipitate was collected by centrifugation at 10 Krpm for 10 minutes. This precipitate was dissolved in 5 ml of 20 mM borate buffer (pH 8.0) and dialysed in the buffer at 5°C overnight.

10 mg of human lysozyme (previously mentioned) was immobilized against Ig of CNBr-activated Sepharose 4B (Pharmacia) according to Manual [Affinity Chromatography, principles & methods, (Pharmacia Fine Chemicals) pI5]. Then anti-human lysozyme antibody was purified using a column filled in with immobilized human lysozyme. Namely, fraction of the above precipitate with ammonium sulfate was put on the column equilibrated with the above-mentioned borate buffer so that antibody would adhere. After rinsing with the above buffer until $A_{280}$ became O, the column was rinsed with 0.I M acetate buffer (pH 4.0) containing 0.5 M NaCl until $A_{280}$ became O. Then, anti-human lysozyme antibody was eluted with 0.2 M glycine-HCl buffer (pH 2.0) containing I M NaCl, and neutralized with I M NaOH after the addition of 0.2 M $Na_2HPO_4$ of I/50 quantity of the eluate.

When IgG was calculated based on an general concept that I mg/ml of IgG shows an $A_{280}$ value of I.5, (I) 2.77 mg, (2) 2.43 mg and (3) 2.83 mg of IgG were obtained from three rabbits [(I), (2) and (3) being identification numbers of rabbits]. Sodium merthiolate (Nakarai Chemicals) was added to the anti-human lysozyme antibody solution to a final concentration of 0.0I% and stored at 4°C. The above procedures were was all manipulated at 5°C.

Example I

Preparation of DNA fragment Coding for Signal Sequence :

As a signal sequence to secrete human lysozyme to a culture medium, a signal peptide of hen egg white lysozyme was used. The nucleotide sequence was determined in consideration of the known amino acid sequence [Jung, A. et al, Proc. Natl. Acad. Sci. 77, 5759 (I980)] and the following points for high expression:

(I) Codons which are highly frequently used for yeast are preferentially selected;

(2) To enhance the expression, a sequence of the yeast PGK gene is used in the upstream of ATG; and

(3) Construction of a hybrid signal is possible.

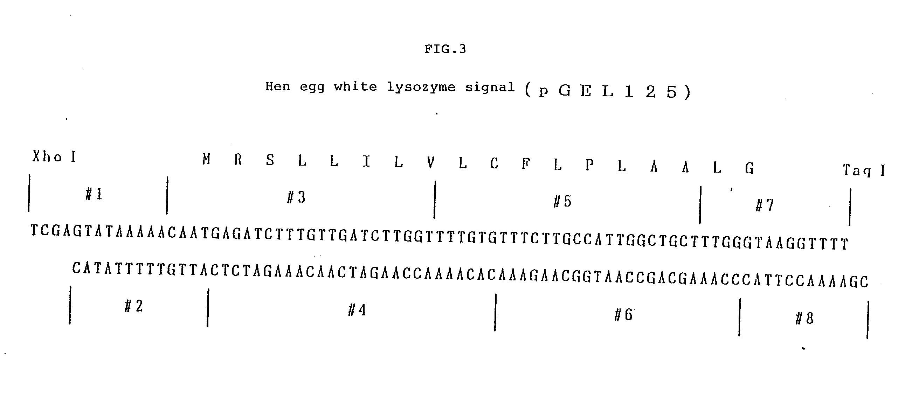

The nucleotide sequence thus synthesized is shown in Fig. 3. As shown, there are provided an XhoI site at the 5'-end and a TaqI site at the 3'-end containing the lysozyme-encoding region. The entire sequence is consisted of eight oligonucleotide blocks (#I-#8) which were prepared by the phosphamide method [Caruthers, M.H. et al; Tetrahedron Letters 22, I859 (I98I)].

The nucleotide blocks #2 to #7 [each I0 μl (5 μg)] were mixed with each other, to which were further added 20 μl of a kinase buffer of a I0-fold concentration (0.5 M Tris-HCl, 0.I M $MgCl_2$, 0.I M mercaptoethanol, pH 7.6), 20 μl of I0 mM ATP, 20 μl (50 u) of T4 polynucleotide kinase (manufactered by Takara Shuzo Inc.) and 80 μl of distilled water. The mixture was then reacted at 37°C for 2 hours and thereafter treated at 65°C for 20 minutes to stop the reaction. To the reaction mixture were added I0 μl (5 μg) of each of the nucleotide blocks #I and #8 and I0 μl of T4 ligase (manufactured by NEB Inc.) and the mixture was reacted at I4°C overnight. The resulting reaction mixture was electrophoresed on I0% polyacrylamide gel. A fragment of 76 bp was cut out of the gel and extracted from the gel by electroelution. This was dissolved in 45 μl of distilled water, to which 6 μl of a kinase buffer of a I0-fold concentration (supra), 6 μl of I0 mM ATP and 2 μl (5 u) of T4 polynucleotide kinase (supra) were added. The mixture was reacted at 37°C for I hour and then stored at -20°C.

In Fig. 3, there is adopted a single letter expression for amino acids (Rule Confirmed by IUPAC-IUB Biochemistry Nomenclature).

Example:

A: Alanine
B: Aspartic acid or Asparagine
C: Cysteine
D: Aspartic acid
E: Glutamic acid
F: Phenylalanine
G: Glycine
H: Histidine
I: Isoleucine
K: Lysine
L: Leucine
M: Methionine
N: Asparagine
P: Proline

O: Glutamine
R: Arginine
S: Serine
T: Threonine
V: Valine
W: Tryptophan
Y: Tyrosine
Z: Glutamic acid or Glutamine
X: Unknown or other amino acids

Example 2

Construction of Secretion Plasmid:

The plasmid pLYS22I (236 μg) obtained in Reference Example 3 was treated with I20 u of EcoRI (manufactured by Nippon Gene Inc.) and I20 u of XhoI (manufactured by Nippon Gene Inc.) at 37°C for 2 hours to cut out a fragment of the human lysozyme-encoding region. The fragment was further treated with 26 u of TaqI (manufactured by Nippon Gene Inc.) at 65°C for I hour to obtain a fragment of the huyman lysozyme encoding region which lacked a portion of the N-terminal portion.

About I μg of the resulting fragment was mixed with 0.5 μg of the signal sequence encoding DNA obtained in Example I and the mixture was reacted in the presence of 800 u of T4 ligase (supra) at I6°C for I6 hours, followed by a treatment with XhoI (42 u).

The thus obtained XhoI fragment (I0 ng) was mixed with I ng of a DNA obtained by treating yeast expression vector pGLD906-I (Japanese Patent Unexamined Publication No. 6I-4399I) with XhoI, and the both was ligated in the presence of T4 ligase.

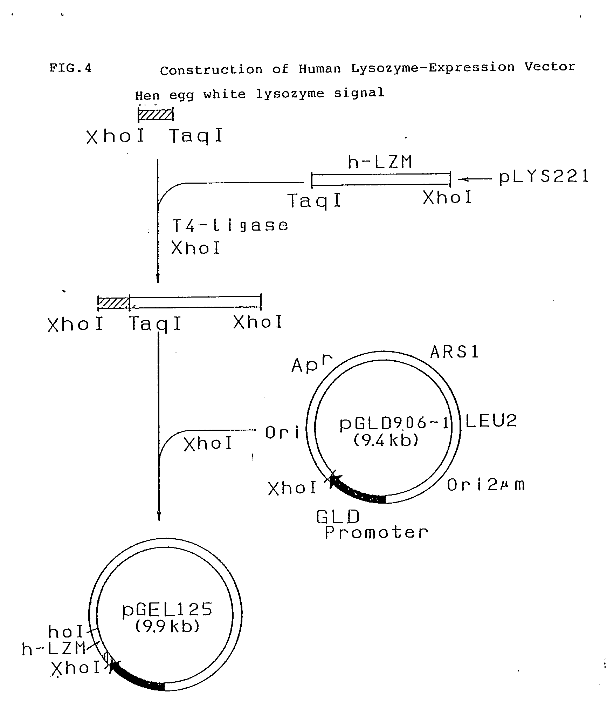

E. coli DHI was transformed with the reaction mixture in the same manner as above to obtain a number of plasmids containing a GLD promoter, downstream of which the signal sequence-encoding region and the human lysozyme gene were inserted in the same direction as that of the promoter. One of such plasmids was named pGELI25 and used in the following tests (see Fig. 4).

Example 3

Preparation of Yeast Transformant:

With the plasmid pGELI25 obtained in Example 2 Saccharomyces cerevisiae AH22R⁻ was transformed in accordance with the method of Hinnen et al (supra) to obtain a transformant Saccharomyces cerevisiae AH22R⁻/pGELI25.

Example 4

Cultivation of the Transformant:

Into a test tube was poured 5 ml of Barkers modified medium I [Amer. J. Bot. 30, 206 (I943); K 3g/l, glucose 30 g/l, asparagine 4 g/l], to which the transformant S. cerevisiae AH22R⁻/pGELI25 was innoculated, and cultivation was carried out at 30°C for 3 days with shaking. One (I) ml of the culture was transferred to another test tube containing 4 ml of the same culture medium and cultivation was carried out at 30°C for one day with shaking. Two (2) ml of the culture was further transferred to a 200 ml flask containing I8 ml of Burkholder modified medium II ($K_2HPO_4$ 0.3g/l, glucose I0 g/l, asparagine 4 g/l, sucrose 50 g/l) and the mixture was cultivated at 30°C for 4 days with shaking. During the cultivation, sampling was made at time of 55, 70 and 80 hours.

Example 5

Preparation of Assay Samples:

The culture obtained in Example 4 was centrifuged to separate a supernatant and cells. The cells were washed with 50 mM phosphate buffer (pH 7.0) containing I.2 M sucrose and then suspended in the same buffer containing 0.5 mg/ml of Zymolyase (manufactured by Seikagaku Kogyo K.K.). The suspension was reacted at room temperature for 2 hours. To the resulting reaction mixture was added 4-fold volume of 50 mM phosphate buffer (pH 7.0) [containing I0 mM EDTA and I mM PMSF] and the mixture was reacted at room temperature for I hour. Thereafter, a supernatant was collected to obtain a cell extract.

Example 6

Measurement of Amount of Human Lysozyme Produced:

The supernatant and the cell extract obtained in Example 5 were assayed for the human lysozyme.
The measurement of the human lysozyme activity was carried out according to Worthigton Enzyme Manual, p. I00, Worthigton Biochemical Corporation, USA, I972. As a standard, human lysozyme manufactured by Sigma Inc. was used. "One unit" is defined as the amount of the enzyme required to reduce the absorbance at 450 m$\mu$ by 0.00l by reaction at 25°C for I minute in 0.I M phosphate buffer (pH 6.9) using Micrococcus lutes (manufactured by Seikagaku Kogyo K. K.) as a substrate. The amount of the human lysozyme produced was as follows:

|  | Human Lysozyme (mg/l) | |
| --- | --- | --- |
| Cultivation time | Supernatant | Cell Extract |
| 55 | 3.1 | 3.4 |
| 70 | 4.0 | 3.2 |
| 80 | 4.3 | 3.5 |

Example 7

Confirmation of Production of Human Lysozyme Using Anti-Human Lysozyme Antibody:

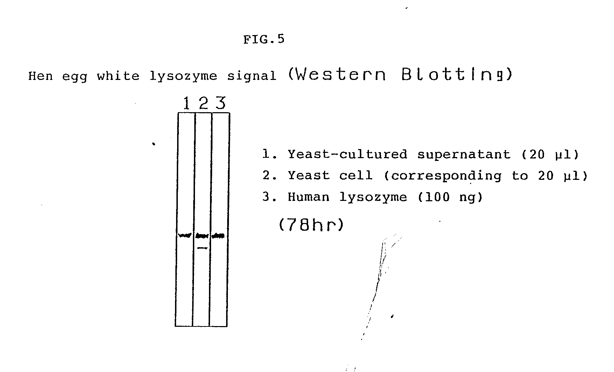

To the culture supernatant or cell extract (each 20 $\mu$l) was added 20 $\mu$l of Laemmli buffer of a two-fold concentration [Laemmli, Nature 227, 680 (I970)] and the mixture was heated at I00°C for 5 minutes. The mixture was then applied to I5% SDS polyacrylamide gel electro-phoresis. Thereafter, the gel was treated by means of a transfer device (manufactured by Bio RAD) at 70 V for 3 hours for transferring the protein from the polyacrylamide gel to a nitrocellulose filter (BA85 manufactured by S & S Inc.). After being blocked with TBS buffer (20 mM Tris-HCl, pH 7.5) containing 3% gelatin, the filter was shaken overnight in a primary antibody solution [TBS buffer containing anti-human lysozyme antibody (see Reference Examples 4 and 5) and I% gelatin]. The detection of the human lysozyme protein was effected with an Immuno-blott assay kit (GAR-HRP of Bio RAD Inc.). As a result, a protein having the same molecular weight as the human lysozyme (Sigma Inc.) derived from human maternal milk was detected both in the supernatant and in the cells (see Fig. 5).

Example 8

Purification and Isolation of Expressed Human Lysozyme:

The transformant S. cerevisiae AH22R⁻/pGELl25 was first cultivated in accordance with the method described in Example 4. Thus, the transformant was cultivated in 1 liter Myer flask containing 250 ml of the medium at 30°C for 70 hours with shaking. The supernatant (4.5 liters) obtained by centrifugation of the culture contained the human lysozyme in an amount of 1,229,000 units (9.45 mg).

(i) Purification by CM-Toyopearl 650C:

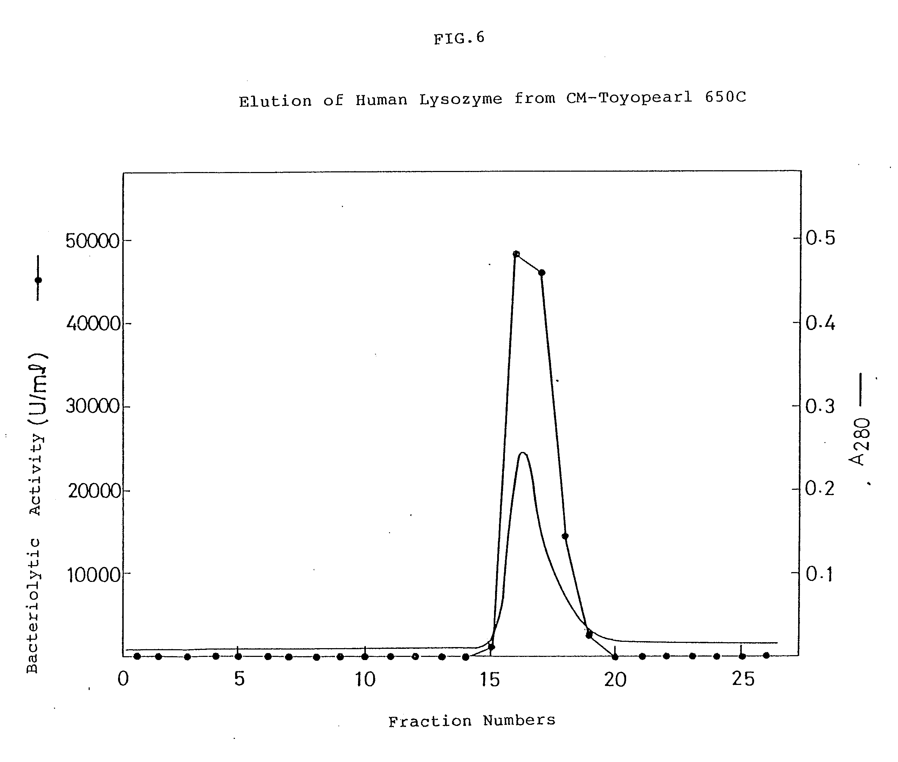

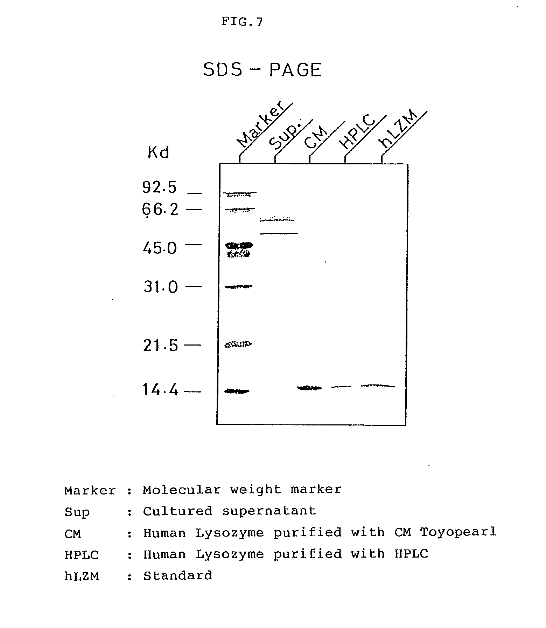

The supernatant thus obtained was adsorbed to CM-Toyopearl 650C colum (∅ 2.6 cm × 34 cm)-equilibrated with 50 mM Na-phosphate buffer (pH 6.5). After rinsing the column with 1.2 liters of the above buffer, the human lysozyme was obtained as a single peak (See Fig. 6) by elution with the above buffer containing 0.5 M NaCl. The peak fraction was applied to SDS-PAGE and stained with Coomassie Blue. As shown in Fig. 7, the human lysozyme was purified to a single substance. The recovery rate was found to be about 100%.

(ii) Purification by HPLC:

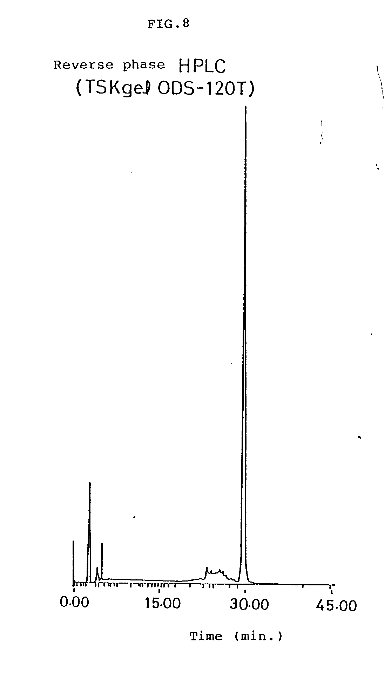

For further purification the human lysozyme obtained in (i) above was applied to HPLC. Thus, 1 ml of the eluate containing 0.2 mg of the human lysozyme was applied on TSK gel ODS 120T. After rinsing with water + 0.1% trifluoroacetic acid (TFA), elution was carried out by a 0-100% gradient method containing $CH_3CN$ in 0.1% TFA to obtain homogeneous human lysozyme (Fig. 8).

Example 9

Properties of Human Lysozyme:

(i) Molecular weight:

The protein obtained in Example 8 was treated with 2-mercaptoethanol and applied to SDS-polyacrylamide gel (15 %) electrophoresis (180 V, 2 hours). A silver staining (a kit manufactured by Daiichi Kagaku Yakuhin) revealed a single band of the protein. This band showed quite the same phoresed distance as that of the human lysozyme specimen electrophoresed simultaneously therewith (Fig. 7). Therefore, the protein was expected to have a molecular weight of 14.7 kd which was identical with the human lysozyme.

(ii) Amino Terminal Amino Acid Sequence :

The protein obtained in Example 8 was subjected to an automated Edman degradation [J. Biol. Chem. 256, 7990 (1981)] using a vapor phase protein sequencer (model 470A manufactured by Applied Biosystem Inc.) to analyze the amino terminal amino acid sequence. The yielded phenylthiohydantoin-amino acids (PTH-amino acids) were identified and quantitatively analyzed by means of high performance liquid chromatography (manufactured by Varian Inc.) using a Micropack SPC 18-3 column to give the results shown in Table below. Thus, it was revealed that the amino acid sequence was H-Lys-Val-Phe-Glu-Arg-X-Glu-Leu-Ala-Arg-(X: UNIDENTIFIED).

| Cycle | | PTH–Amino Acid | |
|---|---|---|---|
| | | (pmole) | (%) |
| 1 | Lys | 816 | 40.8 |
| 2 | Val | 715 | 35.8 |
| 3 | Phe | 812 | 40.6 |
| 4 | Glu | 1113 | 55.7 |
| 5 | Arg | 432 | 21.6 |
| 6. | X * | – | – |
| 7 | Glu | 885 | 44.3 |
| 8 | Leu | 654 | 32.7 |
| 9 | Ala | 633 | 31.7 |
| 10 | Arg | 329 | 16.5 |

* : Unidentified

(iii) <u>Amino Acid Composition</u>:

The protein obtained in Example 8 was placed in a glass tube for hydrolysis and dried under vacuo. Then, 6N HCl or 4% thioglycolic acid-containing 6N HCl was added into the test tube and sealed under vacuo. Hydorlysis was carried out at 110°C for 24 hours. After hydrolysis, the hydrochloric acid was removed under vacuo and the residue was dissolved in 0.02 N HCl to conduct amino acid analysis. The amino acid analytical values were obtained by taking an average of the values obtained for the two kinds of the hydrolyses. However, for tryptophan, the value obtained in the hydrolysis with 4% thioglycolic acid-containing hydrochloric acid was adopted and, for cysteine, the value obtained in the hydrolysis with hydrochloric acid was adopted. The results are shown in Table below.

Amino Acid Composition (*)

| Amino Acid | Analysis Value | Found | Theoretical |
|---|---|---|---|
| Lys | 0.0708(μ mol) | 5.2 | 5 |
| His | 0.0134 | 1.0 | 1 |
| Arg | 0.1946 | 14.2 | 14 |
| Trp | 0.0614 | 4.5 | 5 |
| Asp | 0.2472 | 18.0 | 18 |
| Thr | 0.0705 | 5.1 | 5 |
| Ser | 0.0739 | 5.4 | 6 |
| Glu | 0.1322 | 9.6 | 9 |
| Pro | 0.0248 | 1.8 | 2 |
| Gly | 0.1566 | 11.4 | 11 |
| Ala | 0.1947 | 14.2 | 14 |
| Half Cys | 0.0682 | 5.0 | 8 |
| Val | 0.1206 | 8.8 | 9 |
| Met | 0.0277 | 2.0 | 2 |
| Ile | 0.0689 | 5.0 | 5 |
| Leu | 0.1132 | 8.2 | 8 |
| Tyr | 0.0842 | 6.1 | 6 |
| Phe | 0.0286 | 2.1 | 2 |

* : Calculated with the value for Asp as 18

In the present case, the results of the analysis are in good conformity with the theoretical values except the poor recovery rate of Half Cys. Accordingly, the human lysozyme obtained in the example is considered to be a natural type human lysozyme.

As is apparent from the above Examples, according to the present invention, it is possible to produce a pure human lysozyme protein having quite the same molecular weight, relative activity, amino acid composition and N-terminal amino acid sequence as those of natural human lysozyme.

## Claims

1. A DNA sequence wherein a DNA segment which codes for hen egg white lysozyme signal peptide and which is synthesized based upon codon usage for yeast is bound to the 5' end of a DNA segment coding for human lysozyme.

2. A DNA sequence according to claim I, wherein the DNA segment coding for hen egg white lysozyme signal peptide has a DNA sequence shown in Fig. 3.

3. A DNA sequence according to claim I, which is pGELI25.

4. A cell transformed with the DNA sequence as claimed in claim I or claim 2.

5. The cell according to claim 4, wherein the host cell is yeast.

6. The cell according to claim 4, and being Saccharomyces cerevisial AH22R⁻/pGELI25 (FERM BP-I345).

7. A process for producing human lysozyme, which comprises cultivating the cell as claimed in claim 4, accumulating human lysozyme in the culture and recovering the same.

FIG.1

0 255 233

Group ( I )   Group ( II )   Group ( III )   Group (IV)   Group ( V )

5'  $\underline{U2\text{-}taq}$ $_o\underline{U3}$ $_o\underline{U4}$ $_o\underline{U5}$   $_o\underline{U6}$ $_o\underline{U7}$ $_o\underline{U8}$ $_o\underline{U9}$ $_o\underline{U10}$   $_o\underline{U11}$ $_o\underline{U12}$ $_o\underline{U13}$ $_o\underline{U14}$ $_o\underline{U15}$   $_o\underline{U16}$ $_o\underline{U17}$ $_o\underline{U18}$ $_o\underline{U19}$ $_o\underline{U20}$   $_o\underline{U21}$ $_o\underline{U22}$ $_o\underline{U23}$ $_o\underline{U24}$ $_o\underline{U25}$ $_o\underline{U26}$

3'  L2-taq L3 L4 L5   L6 L7 L8 L9 L10   L11 L12 L13 L14 L15   L16 L17 L18 L19 L20   L21 L22 L23 L24 L25 L26

T4 DNA ligase    T4 DNA ligase    T4 DNA ligase    T4 DNA ligase    T4 DNA ligase

o   5'-terminal phosphoric acid

T4 DNA ligase

Human lysozyme gene

Polynucleotide kinase
ATP

FIG.2

TaqI
```
TCGAGAGATGCGAATTAGCCAGAACTTTGAAGAGATTGGGTATGGACGGCTACCGTGGTATTTC
AGCTCTCTACGCTTAATCGGTCTTGAAACTTCTCTAACCCATACCTGCCGATGGCACCATAAAG
```

                                           HpaII                    HaeIALuI
```
TTTAGCCAACTGGATGTGTCTTGCTAAGTGGGAATCCGGCTATAACACTAGAGCTACCAATTAC
AAATCGGTTGACCTACACAGAACGATTCACCCTTAGGCCGATATTGTGATCTCGATGGTTAATG
```

                                                          XbaI
```
AACGCTGGCGACCGTTCTACAGACTATGGTATTTTCCAAATTAACTCTAGATATTGGTGTAACG
TTGCGACCGCTGGCAAGATGTCTGATACCATAAAAGGTTTAATTGAGATCTATAACCACATTGC
```

                                                          ALuI
```
ATGGCAAGACTCCAGGTGCCGGTCAACGCCTGTCACTTATCTTGCTCAGCTTTGCTTCAGGACAA
TACCGTTCTGAGGTCCACGGCAGTTGCGGACAGTGAATAGAACGAGTCGAAACGAAGTCCTGTT
```

            HhaI
```
CATTGCTGATGCTGTTGCCTGCGGCTAAGAGAGTTGTCCGTGACCCACAGGGTATTAGAGCCTGG
GTAACGACTACGACAACGGACGCGATTCTCTCAACAGGCACTGGGTGTCCCATAATCTCGGACC
```

```
GTCGCTTGGAGAAACAGATGCCAAAATAGAGATGTCAGACAATACGTTCAAGGTTGTGGTGTTT
CAGCGAACCTCTTTGTCTACGGTTTTATCTCTACAGTCTGTTATGCAAGTTCCAACACCACAAA
```

              XhoI
        ALuI
```
AATAGCTCGA
TTATCGAGCT
```

0 255 233

FIG.3

Hen egg white lysozyme signal ( p G E L 1 2 5 )

```
Xho I              M  R  S  L  L  I  L  V  L  C  F  L  P  L  A  A  L  G          Taq I
|      #1      |              #3              |           #5          |     #7      |
TCGAGTATAAAAACAATGAGATCTTTGTTGATCTTGGTTTTGTGTTTCTTGCCATTGGCTGCTTTGGGTAAGGTTTT
    CATATTTTTGTTACTCTAGAAACAACTAGAACCAAAACACAAAGAACGGTAACCGACGAAACCCATTCCAAAAGC
    |     #2     |           #4           |          #6          |       #8       |
```

FIG.4  Construction of Human Lysozyme-Expression Vector

pGEL125 (9.9 kb), pGLD906-1 (9.4 kb)

FIG.5

Hen egg white lysozyme signal (Western Blotting)

1. Yeast-cultured supernatant (20 µl)
2. Yeast cell (corresponding to 20 µl)
3. Human lysozyme (100 ng)

(78hr)

0 255 233

FIG.6

Elution of Human Lysozyme from CM-Toyopearl 650C

FIG.7

# SDS − PAGE

Marker : Molecular weight marker

Sup    : Cultured supernatant

CM     : Human Lysozyme purified with CM Toyopearl

HPLC  : Human Lysozyme purified with HPLC

hLZM  : Standard

FIG.8

Reverse phase HPLC
(TSKgel ODS-120T)

0.00          15.00          30.00          45.00

Time (min.)